# EUROPÄISCHE PATENTANMELDUNG

(11) **EP 0 636 884 A1**
(43) Veröffentlichungstag der Anmeldung: **01.02.1995**
(21) Anmeldenummer: 94110195.8
(22) Anmeldetag: 30.06.1994
(51) Int. Cl.: G01N 33/573, G01N 33/543, G01N 33/577, C12Q 1/34, C12Q 1/37, C12Q 1/40

(54) **Verfahren zur Bestimmung exogener Enzyme in Substraten**

(30) Priorität: 16.07.1993 DE 4323959
(71) Anmelder: ECO SYS Chemische Analysen GmbH, D-76595 Forbach (DE)
(72) Erfinder: Hengerer, Bastian, Dr., D-79650 Schopfheim (DE)
(74) Vertreter: Kolb, Helga, Dr. Dipl.-Chem.

(57) **Zusammenfassung**

Es wird ein Verfahren zur Unterscheidung funktionell vergleichbarer exogener und endogener Enzyme in Substraten beschrieben, welches dadurch gekennzeichnet ist, dass man Antikörper bildet, die auf determinante Stellen am exogenen und/oder endogenen Enzym gerichtet sind, durch die sich das exogene und endogene Enzym voneinander unterscheiden, und mit Hilfe dieser Antikörper qualitativ und/oder quantitativ das endogene und/oder exogene Enzym bestimmt.

Das Verfahren eignet sich zur Überprüfung des exogen einem Substrat zugegebenen Enzyms sowie auch zur laufenden Kontrolle der Enzym-Aktivität während eines Verfahrensschrittes in der Nahrungsmittel- oder Viehfutterindustrie und anderen chemischen oder biologisch-technischen Industriezweigen.

## Beschreibung

Die Erfindung betrifft ein Verfahren zur Unterscheidung funktionell vergleichbarer exogener und endogener Enzyme in Substraten und ein Verfahren zur qualitativen und quantitativen Bestimmung eines exogenen Enzyms, welches in einem Substrat neben einem funktionell vergleichbaren endogenen Enzym vorliegt.

Enzyme sind Biokatalysatoren, welche den Ablauf chemischer Reaktionen erleichtern, so dass diese bei niedrigeren Temperaturen bzw. mit höherer Geschwindigkeit ablaufen. Im allgemeinen erfolgen enzymatische Reaktionen innerhalb eines engen pH- und Temperaturbereiches. Da Enzyme Proteine sind, unterliegen sie bei höheren Temperaturen einem Abbau bzw. einer Denaturierung.

Enzyme setzt man für eine Reihe von industriell durchgeführten Verfahren ein, so z.B. bei der Hydrolyse spezifischer Proteine und Stärken sowie auf anderen Gebieten der Nahrungsmittelindustrie, in der Papierindustrie, der Textilindustrie sowie allgemein in der chemischen Industrie (z.B. in Waschpulvern). Enzyme werden auch für die Behandlung von Abwasser verschiedenen Ursprungs verwendet (z.B. aus der Nahrungsmittelindustrie) wie auch zur Behandlung von Rückständen aus einer Reihe von Verfahren, wobei diese Rückstände vor allem organischer Natur sind (z.B. Schlachthausrückstände).

Man verwendet Enzyme auch zur Unterstützung der Verdauung sowie der Stimulierung anderer Stoffwechselfunktionen bei der menschlichen Ernährung. In der Human- und Tiermedizin verwendet man Enzyme zur Beschleunigung des Heilungsprozesses, zur Eindämmung von Entzündungen etc.. In der Nahrungsmittelindustrie werden Enzyme für eine Reihe von Verfahren eingesetzt, welche die Nahrungsmittelherstellung erleichtern (z.B. in der milchverarbeitenden Industrie). Enzyme dienen auch dazu, für die Tierhaltung das Viehfutter zu behandeln, so dass billigere Ausgangsmaterialien eingesetzt werden können oder eine bessere Resorption gesichert ist.

Zu den vorstehend genannten Zwecken gewinnt man die Enzyme aus Extrakten von Pflanzen, Tierprodukten, Bakterien, Hefen oder Pilzen. Für letztere kann man Bakterien, Hefen oder Pilze in Fermentern unter kontrollierten Bedingungen züchten und daraus die Enzyme gewinnen (z.B. Enzyme aus Trichoderma reesei oder Bacillus subtilis). Durch diese Verfahren erhält man im allgemeinen ein Gemisch aus einer Anzahl von Enzymen, welches dann zu dem jeweiligen Substrat zugegeben wird. In vielen Fällen enthalten die Substrate bereits endogene Enzyme desselben Typs, d.h. Enzyme, die vergleichbare Reaktionen beeinflussen. Die endogenen Enzyme liegen jedoch in der Regel in so geringer Konzentration vor, dass eine ausreichend schnelle und effiziente Reaktion der gewünschten Art nicht abläuft. Aus diesem Grunde ist es erforderlich, exogene Enzyme zuzugeben.

Da, wie vorstehend erwähnt, die Enzyme Biokatalysatoren sind, die zwar an einer bestimmten chemischen Reaktion teilhaben, in derselben aber nicht verbraucht werden, genügen kleine Mengen des Enzyms, um eine verhältnismässig grosse Menge Substrat zu behandeln.

Aus diesem Grunde stellt die Zugabe exogener Enzyme eine sehr kosteneffiziente und umweltfreundliche Massnahme dar, um Nahrungsmittel und Viehfutter zu verbessern und technische Prozesse auf natürliche und effiziente Weise zu beeinflussen.

In den verschiedenen Verarbeitungsprozessen unterliegen die eingesetzten Enzyme den jeweiligen Bedingungen, d.h. sie werden beeinflusst von Temperatur aber auch von Verarbeitungsschritten, wie Mahlen, Extrusion, Pelletierung. Aus diesem Grunde ist es, um eine konstante und optimale Reaktion zu erzielen, vorteilhaft, die Enzymaktivität während des Verarbeitungsprozesses zu kontrollieren. Es ist interessant, in den einzelnen Verarbeitungsstufen die Enzymaktivität zu messen und gegebenenfalls weiteres exogenes Enzym zuzuführen.

Ausserdem besteht heute bei der Registrierung von Arzneimitteln und Futtermitteln ein Bedarf, den Behörden gegenüber den jeweiligen Gehalt an exogenem und endogenem Enzym anzugeben. Hierfür aber stand bisher kein Bestimmungsverfahren zur Verfügung, mit dem Enzyme unterschiedlichen Ursprungs mit der erforderlichen Genauigkeit gemessen werden konnten.

Für die Enzymbestimmung stehen zahlreiche analytische Methoden zur Verfügung, die jedoch in der Regel so adaptiert sind, dass sie am besten mit reinen und verhältnismässig hohen Enzymkonzentrationen durchgeführt werden. Dabei ist es nicht möglich, zwischen exogenen und endogenen Enzymen, die ein und dieselbe Reaktion katalysieren, zu unterscheiden. D.h., die Enzymnachweisverfahren bestimmen die Enzymkonzentration lediglich aufgrund ihrer Aktivität und unterscheiden nicht nach dem jeweiligen Ursprung des Enzyms (z.B. Sarada R., Joseph R.: Profile of hydrolases acting on major macromolecules of tomato processing waste during anaerobic digestion, Enzyme Microb. Technol. 15, S. 339-342, 1993). Enzymbestimmungsverfahren, die jedoch lediglich die Aktivität eines Enzyms messen, ohne dabei zu unterscheiden, ob es sich um ein exogenes oder endogenes Enzym handelt, sind dann nicht geeigner, wenn z.B. die Frage gestellt wird, wieviel exogenes Enzym zu einem Substrat zugegeben wurde, und wieviel von dessen Aktivität während der einzelnen Verarbeitungsschritte oder der Lagerung noch erhalten ist.

Ausserdem ist es auch schwierig, die zum Teil sehr niedrigen Konzentrationen an exogenen Enzymen mit traditionellen Methoden zu identifizieren und zu bestimmen; aie Ergebnisse sind äusserst ungenau und eine Unterscheidung von Quantität und Aktivität exogener und endogener Enzyme ist nicht möglich.

J. Kas et al., Trends in Analytical Chemistry 5 (1986) 8, 205-209 und T. Burianova et al., Chemical Abstracts CA116(9):82299e beschreiben die Bestimmung der Konzentration exogener Enzyme mittels Enzymimmunoassays.

Aufgabe der Erfindung ist es, ein quantitatives und genaues, zuverlässiges Testverfahren zur Bestimmung geringer Konzentrationen exogener Enzyme in verschiedenen Substraten (z.B. Nahrungsmittel und Viehfutter) zur Verfügung zu stellen, um auf diese Weise deren Zugabe zu kontrollieren bzw. zu optimieren.

Diese Aufgabe wird gemäss der Erfindung durch ein Verfahren zur Unterscheidung funktionell vergleichbarer exogener und endogener Enzyme in Substraten mit den, Merkmalen gemäß Anspruch 1 und ein Verfahren zur Bestimmung eines exogenen Enzyms, welches in einem Substrat neben einem funktionell vergleichbaren endogenen Enzym vorliegt, mit den Merkmalen gemäß Anspruch 2 gelöst.

Zweckmäßige Ausgestaltungen dieser Verfahren sind Gegenstand der Ansprüche 3 bis 12.

Das erfindungsgemässe Verfahren erlaubt zuverlässig und kostengünstig die folgenden Bestimmungen:
- die Identifizierung spezifischer Enzyme,
- die Quantifiziereung spezifischer Enzyme,
- die Differenzierung zwischen exogenen und endogenen Enzymen und deren Enzymaktivität, sowie
- die Quantifizierung der spezifischen Aktivität exogener Enzyme bei niedrigen Konzentrationen.

Das erfindungsgemässe Verfahren beschränkt sich dabei nicht auf spezifische Enzyme oder spezifische Substrate.

Die beiliegenden Figuren illustrieren Folgendes:
- Fig. 1: stellt eine Standardkurve dar, die mit Hilfe bekannter Mengen von Roxazym^{T} aufgenommen wurde.
- Fig. 2: ist das Ergebnis des erfindungsgemässen Verfahrens an Viehfutter-Proben, wobei diesem Viehfutter eine bekannte Menge an Roxazym^{T} zugegeben wurde.

Beispiele für den erfindungsgemässen Einsatz stellen dar:
Amylasebestimmung im Brot, Bestimmung von Proteasen bei der Aufarbeitung von Schlachtabfällen für Viehfutter, Bestimmung von Proteasen, Xylanasen und Cellulasen bei der allgemeinen Verbesserung von Viehfutter, Bestimmung von Proteasen in der milchverarbeitenden Industrie wie auch in der lederverarbeitenden Industrie, Bestimmung von Glucanasen in der Brauerei, Bestimmung von Pectinasen bei der Herstellung von Fruchtsäften.

Während die endogenen Enzyme in Abhängigkeit vom Substrat pflanzlichen oder tierischen Ursprungs sind, sind die exogenen Enzyme in der Regel isoliert aus Bakterien-, Hefen- oder Pilzextrakten. Ungeachtet ihres verschiedenen Ursprungs unterscheiden sich Enzyme einer Klasse nicht in der von ihnen katalysierten Reaktion, d.h. Cellulasen pilzlichen Ursprungs oder aus Pflanzen katalysieren in gleicher oder vergleichbarer Weise den Abbau von Cellulose. Für eine Bestimmung der exogen zugegebenen Enzyme ist es daher notwendig, exogene und endogene Enzyme voneinander zu trennen. Ein mechanisches Trennverfahren, z.B. über die Chromatographie, ist aber äusserst zeit- und arbeitsaufwendig und aufgrund der geringen Mengen nur äusserst schwierig durchführbar. Es ist daher erforderlich, ein Verfahren zur Verfügung zu stellen, welches die Enzyme unterschiedlicher Herkunft erkennt und eine Bestimmung von exogenem und endogenem Enzym erlaubt.

Die erfindungsgemässe Bestimmungsmethode, nach der exogene und endogene Enzyme voneinander getrennt bestimmt werden können, basiert auf dem Einsatz von Antikörpern, die determinante Stellen (Epitope) am jeweiligen exogenen und/oder endogenen Enzym erkennen und an diese binden. Diese determinanten Stellen müssen dabei so gewählt werden, dass sich exogenes und endogenes Enzym voneinander unterscheiden.

Ein Vergleich der Aminosäuresequenz von Enzymen, die unterschiedlicher Herkunft sind, aber die gleiche Enzymreaktion katalysieren, haben ergeben, dass zahlreiche Abweichungen bestehen und die Homologie, d.h. die Übereinstimmung der beiden Aminosäuresequenzen in vielen Fallen unter 50% und zum Teil weit darunter liegt. Bei einer derart geringen Homologie ergibt sich zwangsläufig eine andere Faltung des Enzymmoleküls und die Bildung anders gearteter determinanter Stellen. Dies wiederum bedeutet, dass selbst ein Gemisch polyclonaler Antikörper, das z.B. gegen ein bakterielles Enzym einer bestimmten Klasse gerichtet ist, sich grundlegend von einem Gemisch polyclonaler Antikörper, welche gegen ein vergleichbares tierisches Enzym gerichtet sind, unterscheiden.

Die vorstehenden Beobachtungen wurden bestätigt durch eine Reihe von Recherchen in Gen-Datenbanken, welche sowohl die DNA-Sequenz als auch die Aminosäuresequenz wiedergeben. Insgesamt sind bis heute 200.000 Gene aus verschiedenen Organismen sequenziert und in Datenbanken niedergelegt. Um gemäss der Erfindung ein geeignetes Testsystem zu entwickeln, kann man z.B. von einer bestimmten bakteriellen oder pilzlichen Cellulase, Glucanase, etc. ausgehen, von der bekannt ist, dass ihre DNA- und Aminosäuresequenz in der Genbank vorliegt. Mit Hilfe eines geeigneten Computer-Programmes ist es dann möglich, innerhalb der Genbank nach solchen Sequenzen zu suchen, die z.B. über eine 50%ige Homologie mit dem bekannten bakteriellen Enzym verfügen. Findet man unter den ermittelten Sequenzen keine pflanzliche oder tierische Glucanase, so hat man bei der hohen Anzahl von Genen, über die die Datenbank verfügt, bereits einen guten Anhaltspunkt dafür, dass das entsprechende pflanzliche oder tierische Enzym über eine geringere Homologie als 50% verfügt. Dies beruht natürlich auf der Annahme, dass die entsprechende Glucanase pflanzlichen oder tierischen Ursprungs ebenfalls in der Datenbank vorliegt.

Der vorstehend gewonnene Anhaltspunkt ist aber ausreichend, um ein Experiment zu rechtfertigen, um die vorstehend gemachte Annahme experimentell zu bestätigen. Für dieses Experiment immunisiert man dann jeweils ein Tier mit dem bakteriellen und z.B. dem tierischen Enzym. Die gebildeten polyclonalen Antikörper werden dann eingesetzt, um mit einem Gemisch bekannter Gehalte an exogenem und endogenem Enzym die Bestimmung experimentell durchzuführen. Lassen sich im immunologischen Test exogenes und endogenes Enzym mit aureichender Genauigkeit bestimmen, so verfügt man über ein Nachweissystem, welches im folgenden für den entsprechenden Enzymtyp auch an Gemischen unbekannter Konzentration eingesetzt werden können. Um Temperatur, Konzentrationseinflüsse, etc. bei dem immunologischen Test auszuschalten, ist es bevorzugt, bei jeder Bestimmung eine Standardkurve für bekannte Enzymkonzentrationen aufzustellen.

Sofern der Einsatz polyclonaler Antikörper keine ausreichend akkurate Bestimmung erlaubt, ist es bevorzugt, monoclonale Antikörper zu entwickeln, die jeweils auf eine oder mehrere bestimmte determinante Stellen im exogenen Enzym gerichtet sind, wobei Voraussetzung ist, dass das endogene Enzym nicht über vergleichbare determinante Stellen verfügt.

Sobald feststeht, dass eine bestimmte Klasse von Enzymen unterschiedlicher Herkunft mit Hilfe von entweder polyclonalen oder monoclonalen Antikörpern unterschieden werden kann, ist es ohne weiteres möglich, einen immunologischen Test durchzuführen. Für diesen immunologischen Nachweis stehen beliebige Testmethoden zur Verfügung. Nach einer bevorzugten Variante wird z.B. der Antikörper immobilisiert. Dies kann mit Hilfe eines geeigneten Puffersystems durch Bindung an eine Mikro-Titerplatte geschehen oder durch Einsatz magnetischer Partikel, an welche die Antikörper gebunden werden. Wenn man dann den immobilisierten Antikörper in Kontakt mit der zu untersuchenden Probe bringt, so binden an diesen jeweils ausschliesslich oder bevorzugt die exogenen oder endogenen Enzyme. Daraufhin werden der Überstand und nicht gebundenes Material durch Waschen entfernt. Sofern die Antikörper-Antigen-Bindung nicht das aktive Zentrum beeinflusst, kann durch Zugabe der entsprechenden Nachweiskomponenten z.B. ohne weiteres in einer Farbreaktion die Aktivität des gebundenen Enzyms (Antigen) bestimmt werden.

Abgesehen von der vorstehend skizzierten Methode ist es aber auch möglich, jedes andere Nachweissystem einzusetzen.

### Allgemeines Anwendungsbeispiel

1. Bestimmung mikrobieller Enzymaktivität, wie (1-3)(1-4)-β-D-Glucanase, (1-4)-β-Glucanase, Pectinase, Xylanase oder Amylase in Substaten, wie Viehfutter:
   Die Enzymaktivitäten werden bestimmt, wie dies von der 'Commission of Biotechnology (Commission of Biotechnology, Int. Union of Pure and Applied Chemistry, in 'Measurement of cellulase activity', T.K. Ghose, E.D., Biochemical Engineering Research Center, Indian Institute of Technology, New Delhi, India, 1984) beschrieben ist.
2. Herstellung spezifischer Antikörper, welche gegen Schlüsselenzyme gerichtet sind, und Koppeln dieser Antikörper an einen festen Träger, wie Magnetteilchen oder Mikro-Titerplatten.
   Zur Immunisierung werden angereicherte oder hochreine Enzyme bzw. Fragmente derselben in Versuchstiere (Kaninchen, Ziegen, Schafe, etc.) injiziert. Nach dem Boosten der Immunantwort wird den Tieren Blut entnommen und es werden die Antiseren gesammelt. Antikörper vom IgG-Typ können nach bekannten Methoden aus dem Serum gereinigt werden.
   Für die Bestimmung ist es bevorzugt, die Antikörper an einen festen Träger zu koppeln. Als solche Träger eignen sich Protein A-Beads, PVC-Mikro-Titerplatten oder magnetische Teilchen. Letztere eignen sich besonders gut, da sie über eine hohe Bindungskapazität verfügen und das Antigen schnell absorbieren.
   Alternativ können spezifische Antikörper gegen ein Enzym, wie 1,4-β-D-Glucanase von Trichoderma reesei gebildet werden. Dieses Enzym umfasst einen katalytischen Bereich und eine spezifische Bindungsdomäne, die durch eine Linkersequenz getrennt sind. (Diese Linkersequenz zeigt keine biologische Aktivität. Die meisten pilzlichen und bakteriellen Enzyme mit Glucanase-Aktivität zeigen solche Strukturmerkmale.) Die Computer-Analye der Peptidstruktur deutet auf ein stark hydrophiles Verhalten sowie auf eine starke Antigenizität eines 10-mer Peptids aus dieser Linkersequenz. Diese Peptidsequenz kann für die Bildung von Antikörpern verwendet werden, wobei eine grosse Wahrscheinlichkeit dahingehend besteht, dass diese Antikörper die enzymatische Aktivität nicht inhibieren werden. Ein solcher Antikörper eignet sich dann besonders für ein simples Nachweissystem, wobei - wie vorstehend bereits erwähnt - dem Antikörper-Antigen-Komplex lediglich die Nachweiskomponenten zugegeben werden und die Enzymaktivität in einer Farbreaktion bestimmt wird.
3. Immunaffinitätsreinigung von Schlüsselenzymen bei bekannten Mengen exogener Enzyme und bei Substraten, denen diese exogenen Enzyme zugegeben wurden.
   Zur Quantifizierung wird das zu analysierende Material in einem Antikörper-Bindungspuffer homogenisiert und dann mit den Antikörpern inkubiert. Bekannte Mengen der Schlüsselenzyme werden in parallelen Reaktionen mit immobilisierten Antikörpern inkubiert, um das System zu kalibrieren.
4. Bestimmung
   a) gereinigter Enzym-Aktivitäten in Standard-Assays. Die Enzym-Aktivität wird wie in 1. beschrieben bestimmt.
   b) Quantitative Bestimmung in einem ELISA-Test oder durch Korrelation der enzymatischen Aktivität mit dem Gewicht einer reinen exogenen Enzympräparation.

   Nach b) wird das antikörpergebundene Enzym mit einem zweiten Antikörper inkubiert, welcher ein unterschiedliches Epitop am gleichen Antigen (Enzym) erkennt. Dieser Antikörper ist kovalent mit einem Enzym gebunden, wie z.B. alkalische Phosphatase, die dann in einem einfachen Test bestimmt werden kann.
   Bevorzugt ist der Einsatz immobilisierter Antikörper, nämlich von Antikörpern, die an einen festen Träger gebunden sind. Auf diese Weise können in einem Routinetest Antigene um den Faktor 1000 bis 100000 gereinigt werden. Dies bedeutet, dass die Enzymbestimmung in Substaten mit sehr geringer Enzym-Konzentration möglich ist.

### Spezielles Ausführungsbeispiel

Das folgende Beispiel zeigt die Anwendung der erfindungsgemässen Methode zur Bestimmung der Cellulase-Aktivität (quantitative Bestimmung) in einer kommerziell erhältlichen Enzym-Präparation (Roxazym^{T} von Hoffman LaRoche), welche einem kommerziell erhältlichen Hühnerfutter zugegeben wurde. Roxazym^{T} enthält verschiedene Cellulasen und Glucanasen.

Zunächst wurde ein polyclonales Antiserum gegen Roxazym^{T} gewonnen, indem Mäuse mit 100 µg Roxazym^{T} in 100 µl physiologischer Kochsalzlösung und 100 µl Freundschem Adjuvans immunisiert wurden. Die Versuchstiere wurden jede dritte Woche geboostet. Daraufhin wurde den Versuchstieren 10 Tage nach den Injektionen Blut aus der Schwanzvene entnommen, um den Antikörper-Titer zu bestimmen. Dazu wurden 10 µg Roxazym^{T} auf einem 12%igen SDS-Polyacrylamid-Gel aufgetrennt und mit Hilfe von Halb-Trocken-Blotting auf eine PVDF-Membran transferiert. Die Membran wurde dann mit dem Antikörper über Nacht inkubiert. Die gebundenen Antikörper wurden mit Hilfe eines zweiten, mit alkalischer Phosphatase markierten Antikörpers und einem chemilumineszenten Substrat (AMPPD) nachgewiesen. Das Blotting und der Nachweis wurden nach den Anweisungen des BIO-RAD Immun-Light-II Kit (Immun-Lite II Assay Kit, BIO-RAD Laboratories, Inc. European Headquaters, Dreve du Senechal, 19, B-11800 Brüssel) bestimmt. Die Antikörper zeigten keine Kreuzreaktion mit Kontrollsubstrat-Proteinen. Es wurde eine starke Bindung mit der Enzym-Präparation Roxazym^{T}, welche für die Immunisierung verwendet wurde, festgestellt.

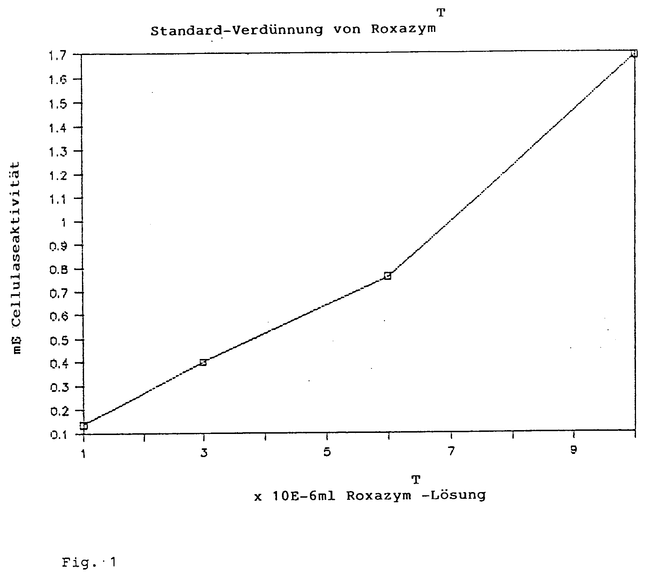

10 µl Antiserum, welches wie vorstehend erhalten wurde, wurden mit 100 µg anti-Maus-IgG-Antikörper (gekoppelt an magnetische Teilchen - BioMag, Magnetic Goat anti-Maus-IgG, Advanced Magnetic Inc, Cambridge, MA) gekoppelt. 100 µg der zu untersuchenden Probe (Hühnerfutter, z.B. KLIBA Mühlen, Klingentalmühle AG, Kaiseraugst/Schweiz) wurden in Bindungspuffer (PBS, zuzüglich 0,4 M NaCl, 1% BSA, 0,05% Tween 20) homogenisiert und zu dem Antikörper-beschichteten System zugegeben. Bei Aufstellung einer Standardkurve (Fig. 1) wurden Verdünnungen bekannter Mengen an Roxazym^{T} in parallelen Ansätzen zugegeben und bestimmt. Nach Inkubation bei 4°C über Nacht unter konstantem Rühren waren die Antigene quantitativ von den Antikörpern gebunden. Die magnetischen Teilchen wurden mit einem starken Magneten gesammelt und die nicht gebundenen Proteine einschliesslich der Enzymhomologen im Substrat in drei Waschschritten mit jeweils 1 ml Waschpuffer (PBS, zusätzlich 1% BSA und 0,05% Tween 20) ausgewaschen. Die Enzyme lagen nun in gereinigter Form vor, und die Aktivität konnte direkt bestimmt werden. 1,4-β-D-Glucanase wurde dabei bestimmt durch Resuspendieren der magnetischen Teilchen, welche entweder die Enzyme aus der zu untersuchenden Probe (Hühnerfutter) oder der Standardverdünnungen bekannter Konzentrationen an Roxazym^{T} enthielten, in 200 µl 25 mM Natrium-Citrat-Puffer, welcher 1% Carboxymethylcellulase (CMC) enthielt, (pH 4,8). Nach 30-minütiger Inkubation bei 50°C wurde die Glucosekonzentration mit Hilfe eines entsprechenden Bestimmungs-Kits von Boehringer Mannheim bestimmt. Dabei wird eine Einheit Cellulase-Aktivität definiert als die Enzym-Aktivität, welche pro Minute 0,18 mg Glucose freisetzt

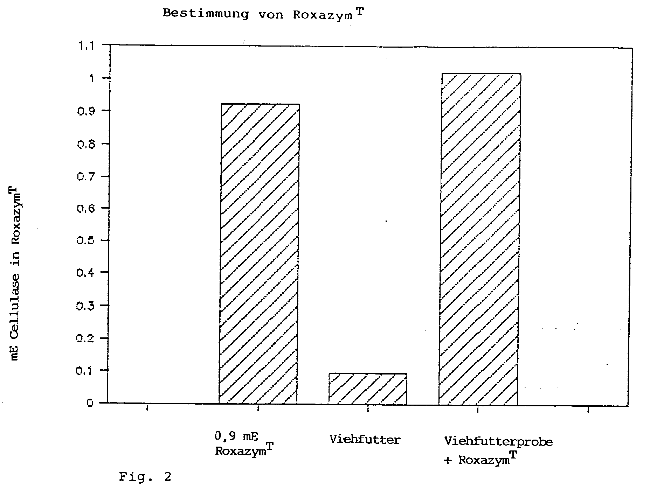

Die Standardkurve in Fig. 1 zeigt einen Korrelationsfaktor von 0,97, was eine hohe Präzision des Testes bedeutet. Die Bestimmung von zugegebenem Roxazym^{T} (bekannte Roxazym^{T}-Menge) zu der Probe, welche eine bekannte Cellulase-Aktivität enthielt, ist in Fig. 2 wiedergegeben.

Die analysierte Roxazym^{T}-Probe ergab eine Aktivität von 140 Einheiten Cellulase-Aktivität/ml. Der zu analysierenden Probe an Viehfutter, welche bereits 0,096 mE endogene Cellulase-Aktivität enthielt, wurden 0,924 mE Roxazym^{T} zugegeben. Die Gesamt-Cellulase-Aktivität wurde zu 1,02 mE bestimmt.

Die analysierten Viehfutter-Proben von KLIBA Mühlen AG enthielten 3750 E, 1507 E, 3140 E, 0 E, 785 E und 4870E exogene Cellulase-Aktivität pro kg Viehfutter.

Die vorstehenden Ergebnisse zeigen, dass das erfindungsgemässe Verfahren gut zwischen exogenen Enzymen (wie Roxazym^{T}) und endogenen Enzymen (bereits im Viehfutter enthalten) quantitativ zu unterscheiden vermag.

Das Verfahren ist dabei sensitiv genug, um die Enzym-Aktivität in hohen Verdünnungen quantitativ in einer Testprobe zu bestimmen.

## Patentansprüche

1. Verfahren zur Unterscheidung funktionell vergleichbarer exogener und endogener Enzyme in Substraten, bei dem man
- Antikörper bildet, die auf determinante Stellen am exogenen und/oder endogenen Enzym gerichtet sind, durch die sich das exogene und endogene Enzym voneinander unterscheiden, und
- mit Hilfe dieser Antikörper qualitativ und/oder quantitativ das endogene und/oder exogene Enzym bestimmt.

2. Verfahren zur Bestimmung eines exogenen Enzyms, welches in einem Substrat neben einem funktionell vergleichbaren endogenen Enzym vorliegt, bei dem man
- Antikörper bildet, die auf solche determinante Stellen am exogenen Enzym gerichtet sind, durch die sich das exogene Enzym von dem endogenen Enzym unterscheidet, und
- mit Hilfe dieser Antikörper qualitativ und/oder quantitativ das exogene Enzym bestimmt.

3. Verfahren nach Anspruch 1 oder 2, dadurch **gekennzeichnet**, daß die Substrate Nahrungsmittel oder Futtermittel oder anderes organisches Material darstellen.

4. Verfahren nach Anspruch 1 oder 2, dadurch **gekennzeichnet**, daß die Antikörper polyclonale Antikörper sind.

5. Verfahren nach Anspruch 1 oder 2, dadurch **gekennzeichnet**, daß die Antikörper monoclonale Antikörper sind.

6. Verfahren nach einem der Ansprüche 1 bis 5, dadurch **gekennzeichnet**, daß die Antikörper in immobilisierter Form vorliegen.

7. Verfahren nach Anspruch 6, dadurch **gekennzeichnet**, daß die Antikörper an magnetische Teilchen gebunden sind.

8. Verfahren nach einem der Ansprüche 1 bis7, dadurch **gekennzeichnet**, daß die Enzymbestimmung in einem Immuntest erfolgt.

9. Verfahren nach einem der Ansprüche 1 bis 8, dadurch **gekennzeichnet**, daß die Antikörper eingesetzt werden, die nach Bindung an das Enzym nicht dessen Aktivität beeinflussen.

10. Verfahren nach Anspruch 9, dadurch **gekennzeichnet**, daß die Aktivitätsbestimmung des Enzyms mit Hilfe eines Farbtestes durchgeführt wird.

11. Verfahren nach einem der Ansprüche 1 bis 10, dadurch **gekennzeichnet**, daµ Cellulasen, Glucanasen, Xylanasen, Proteasen, Pectinasen und Amylasen bestimmt werden.

12. Verfahren nach einem der Ansprüche 1 bis 10, dadurch **gekennzeichnet**, daß exogene Enzyme während eines Produktionsprozesses bestimmt werden.
